# EUROPEAN PATENT APPLICATION

(11) **EP 2 832 849 A1**
(43) Date of publication of application: **04.02.2015**
(21) Application number: 13770215.5
(22) Date of filing: 26.03.2013
(51) Int. Cl.: C12N 1/20, C01B 25/01, C12R 1/01

(54) **ADSORBENT AGENT, COMPOSITION FOR BIOFLOTATION AND APATITE-QUARTZ SYSTEM BIOFLOTATION PROCESS**

(30) Priority: 28.03.2012 BR 102012009197
(71) Applicant: Facultades Católicas, Associação Sem Fins Lucrativos, Mantenedora da Pontificia Universidad Catolica do Rio de Janeiro -Puc Rio, 22451-900 Rio de Janeiro - RJ (BR)
(72) Inventor: TOREM, Maurício Leonardo, CEP: 22451-900 Rio de Janeiro - RJ (BR); MERMA, Gutiérrez Antonio, CEP: 22451-900 Rio de Janeiro - RJ (BR)
(74) Representative: Herzog, Fiesser & Partner Patentanwälte PartG mbB
(86) International application number: PCT/BR2013/000093
(87) International publication number: WO 2013/142933

(57) **Abstract**

The present invention discloses the use of *Rhodococcus opacus* bacteria for bioflotation of minerals of the apatite-quartz system, by adsorption on their surface and subsequent changing of the zeta potential, making them hydrophobic, in addition to reducing the surface tension of water.

## Description

### Field of the Invention

The present invention relates to microorganisms for adsorption in minerals and a process of bioflotation and separation thereof, particularly the flotation of quartz.

### Background of the Invention

Phosphorus, along with nitrogen and potassium, make up the group of primary macronutrients, essential elements for the development of plants. It is important to consider that Phosphorus has no substitutes in agriculture, is not found in a pure state in nature and, in combination with various elements, forms a large variety of compounds. The deposits of phosphatic rocks are the most important sources of this element, having igneous, sedimentary, metamorphic source or resulting from accumulation of organic matter coming from poultry manure.

The main mineral from phosphatic rocks is apatite, the largest importance thereof being related to the production of fertilizers and phosphoric acid which can be produced by wet methods with the use of sulfuric acid. Phosphoric acid is a chemical compound of great importance used in the production of fertilizers, such a Simple Superphosphate-SSP (Ca(H₂PO₄)2.H₂O + CaSO₄), Triple Superphosphate-TSP (Ca(H₂PO₄)₂), Mono-ammonic Phosphate-MAP ((NH₄)H₂PO₄), Diammonic Phosphate-DAP ((NH₄)₂HPO₄) in addition to NP and NPK fertilizers.

In order to meet the increasingly restricted specifications of the market, the phosphate producer has the need to reduce operational costs; to achieve this, research and development of new operational conditions of beneficiation have been encouraged. In addition to this search for cost reduction, the phosphate producer has been facing a major problem, the exhaustion of the deposits with suitable levels of the above-mentioned elements. In this regard, more complex processing of phosphate ore has become increasingly necessary, leading to the development of new equipment, techniques and reagents for the mineral concentration.

Flotation is the most used process to treat phosphate ore, thanks to the easy separation that can exist between phosphates and gangue minerals-gangue minerals associated with apatite are mainly fluorides, carbonates, clays, quartz silicates and metallic oxides - however, with changes in mineralogical composition the result would certainly be different. These changes can be caused when raw material is modified, which happens when complex ores are used, where the presence of gangue minerals is greater, and when different types of apatite are processed. Thus, changes in mineralogical composition cause the mineral to react differently to flotation processes. Many studies have been carried out to obtain low cost reagents, without compromising the selectivity, and that are able to achieve satisfactory or desired recovery values.

The use of micro-organisms in mineral processing, as well as the remediation of waste in mineral industry has aroused great interest and has become an increasingly studied and explored field of biotechnology, and a new proposal for the use of micro-organisms is their application as reagents for flotation. The presence of certain ionizable functional groups in microbial surface gives the microorganisms certain adsorption characteristics which make them capable of replacing certain conventional chemical reagents for flotation and flocculation in mineral processing operations.

US 1,914,694 describes the concentration of phosphatic materials by the method of flotation of phosphatic minerals associated with quartz from gangue minerals. The same does not mention the use of biological adsorbent agents in the flotation process.

US 2,384,825 describes the method of separation of sand, silt and the like from low degree phosphatic rock from wash waste known as debris. The same does not mention the use of biological adsorbent agents in the flotation process according to the present invention.

US 3,534,854 describes the method of separation of calcite and apatite particles, or the like, by flotation in an aqueous solution with high pH. The document cites quartz mineral as gangue mineral contaminant of calcite, however, the same informs that this will be separated from calcite along with apatite, i.e., it does not suggest a method for separating the system composed of quartz and apatite minerals. The same does not mention the use of biological adsorbent agents in the flotation process.

Smith et al. [Recents Developments in the Bioprocessing Minerals. Mineral Processing and Extractive Metallurgy Review, 1993, v. 12, 37] discloses the potential use of micro-organisms for the bioprocessing of minerals, applications such as bio-oxidation of minerals, heavy metal removal, flocculation and flotation of minerals.

Mesquita et al. [Biobeneficiamento mineral: potencialidades dos microrganismos como reagentes de flotação. Série Tecnologia Mineral CETEM, 2002, 81] discloses the potential use of micro-organisms, as well as bacteria of the *Rhodococcus* genus, as agents of flotation of various mineral systems, by adsorption on the surface of minerals and changing of the zeta potential thereof.

Mesquita et al. [Interaction of a hydrophobic bacterium strain in a hematite-quartz flotation system. International Journal of Mineral Processing, 2003, 71] shows the potential of the use of the species of bacterium *Rhodococcus opacus* as mineral agent adsorbent for the flotation of the hematite-quartz system.

However, it is evident by the documents above that there is still a need to study the process of bioflotation for the apatite-quartz system, recalling that the apatite is the most abundant phosphatic ore and quartz is the main gangue mineral associated with it. A high efficiency system for the obtaining of these ores is still required.

### Brief Description of the Invention

The present invention solves the problem of separation of mineral constituents of the apatite-quartz system, through a new biological adsorbent agent for the bioflotation process. The process occurs through the use of bacteria of the genus *Rhodococcus* as adsorbent agent for the surface of the apatite mineral, making it hydrophobic and allowing its separation by flotation.

In a preferred embodiment, the bacterium of the genus *Rhodococcus* comprises the species *Rhodococcus opacus.*

In a preferred embodiment, the bacterium *Rhodococcus opacus* comprises an adaptation by being previously exposed to minerals, in order to increase its affinity thereto, during the process of bioflotation.

In a preferred embodiment, the apatite mineral comprises the fluorapatite subgroup.

The present invention additionally relates to the use of a composition for the process of bioflotation of the apatite quartz system comprising:
a. at least one adsorbent agent comprising at least a bacterium selected from the group comprising the species of the genus *Rhodococcus;* and
b. an acceptable carrier.

In a preferred embodiment, the bacterium of the genus *Rhodococcus* comprises the species *Rhodococcus opacus.*

In a preferred embodiment, the bacterium *Rhodococcus opacus* is a bacterium adapted by being previously exposed to minerals, in order to increase its affinity thereto, during the process of bioflotation.

In a preferred embodiment, the apatite mineral comprises the fluorapatite subgroup.

In a preferred embodiment, the acceptable carrier comprises a support. In a preferred embodiment, the support comprises a saline solution.

In a preferred embodiment, the saline solution comprises a solution of sodium chloride (NaCl).

It is, additionally, an object of the present invention the process of bioflotation of the apatite-quartz system, comprising the steps of:
a. adding the apatite-quartz system in an adsorbent composition comprising at least one adsorbent agent comprising at least a bacterium selected from the group comprising the species of the genus *Rhodococcus;*
b. allowing the adsorbent agent to contact the solution comprising the apatite-quartz system in a Hallimond tube, for flotation during two minutes; and
c. collecting the floated mass comprising quartz and the adsorbent agent.

In a preferred embodiment, the bacterium of the genus *Rhodococcus* comprises the species *Rhodococcus opacus.*

In a preferred embodiment, the bacterium *Rhodococcus opacus* is a bacterium adapted by being previously exposed to minerals, in order to increase its affinity thereto, during the process of bioflotation.

In a preferred embodiment, the apatite mineral comprises the fluorapatite subgroup.

These and other objects of the invention will be immediately valued by persons skilled the art and by companies with interests in the segment, and will be described in sufficient detail for their reproduction in the following description.

### Brief Description of Figures

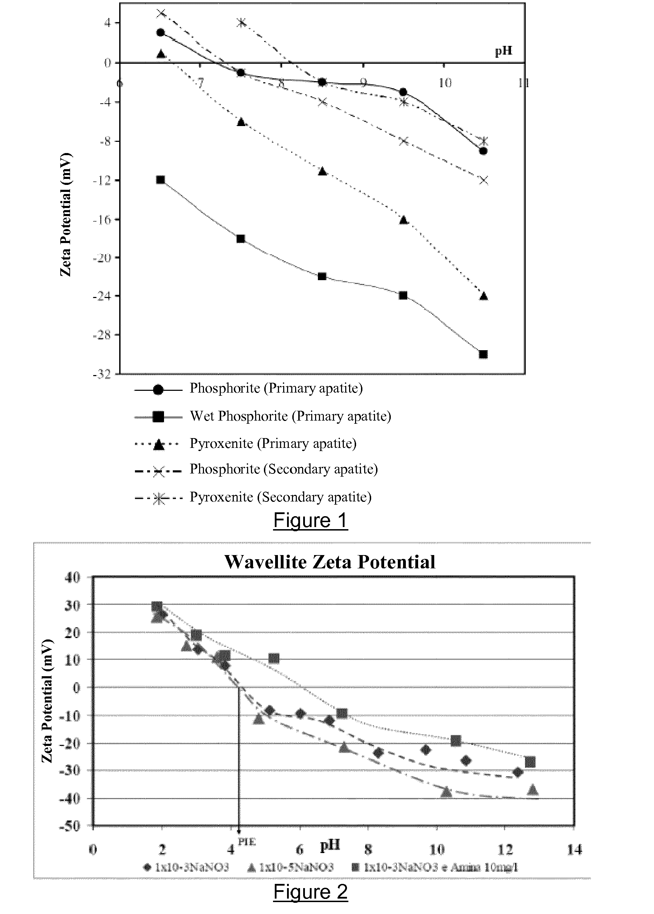

- Figure 1 discloses the zeta potential of apatites and gangue mineral as a function of pH, thus deducting, their isoelectric point PIE.
- Figure 2 discloses the zeta potential as a function of pH of Wavellite, derived from an isomorphic substitution of apatite.

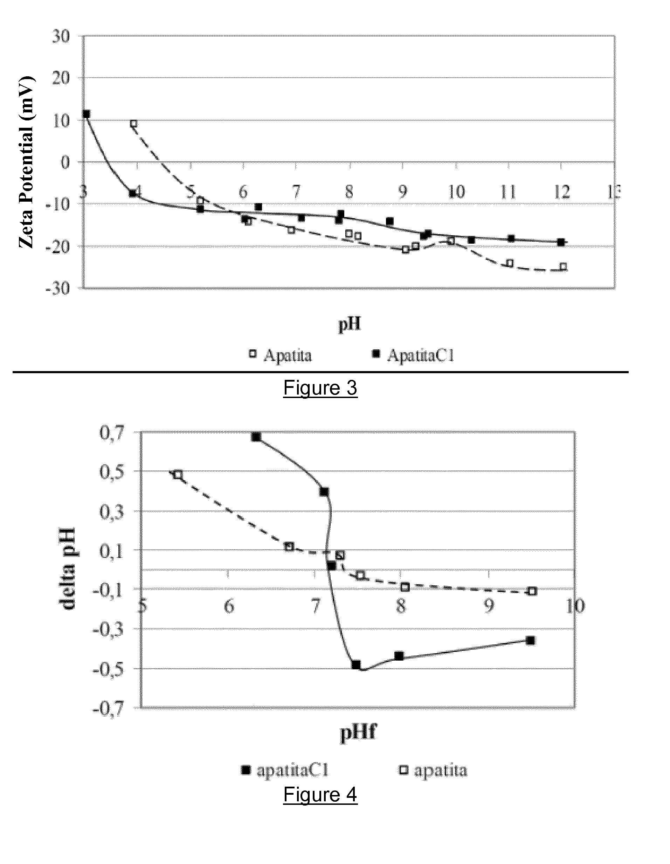

- Figure 3 discloses the zeta potential as a function of pH of apatites, a different determining method, the Dos Santos method.
- Figure 4 discloses the condition of zero charge for apatites, for the calculation of the isoelectric point by the Mular & Roberts method.

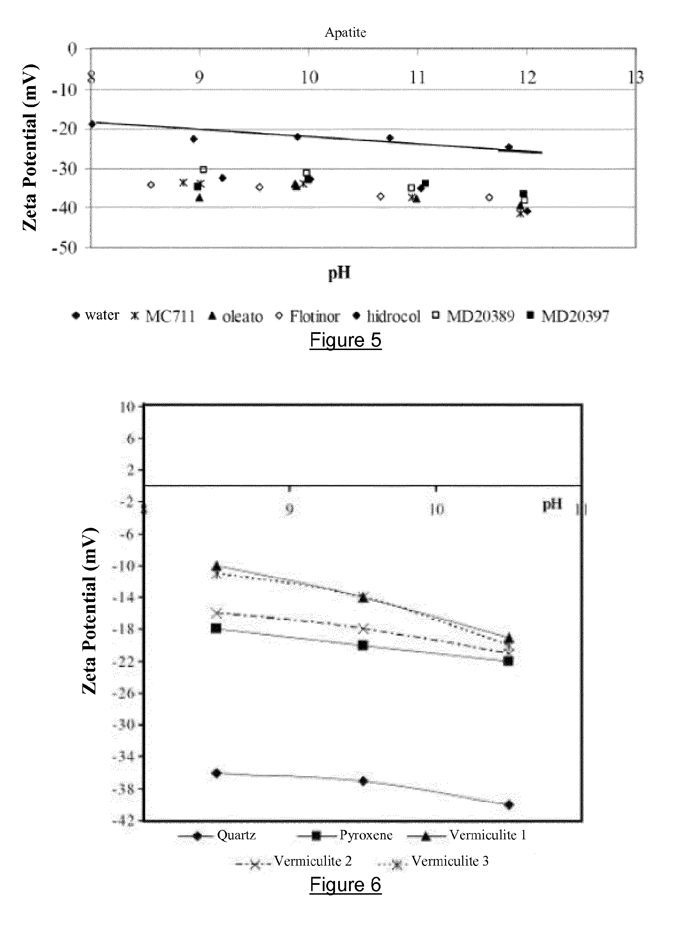

- Figure 5 discloses the zeta potential as a function of pH of apatites in the presence of anionic collectors.
- Figure 6 discloses the zeta potential as a function of pH for apatites and gangue minerals.
- Figure 7 discloses the zeta potential as a function of pH of hematites and quartz.
- Figure 8 discloses a micrograph of the bacterium *Rhodococcus opacus* performed by scanning microscopy.
- Figure 9 discloses an infrared spectrogram of the bacterium *Rhodococcus opacus* and its main peaks of absorbance.
- Figure 10A discloses the results of adhesion in protein adsorption in mineral samples.

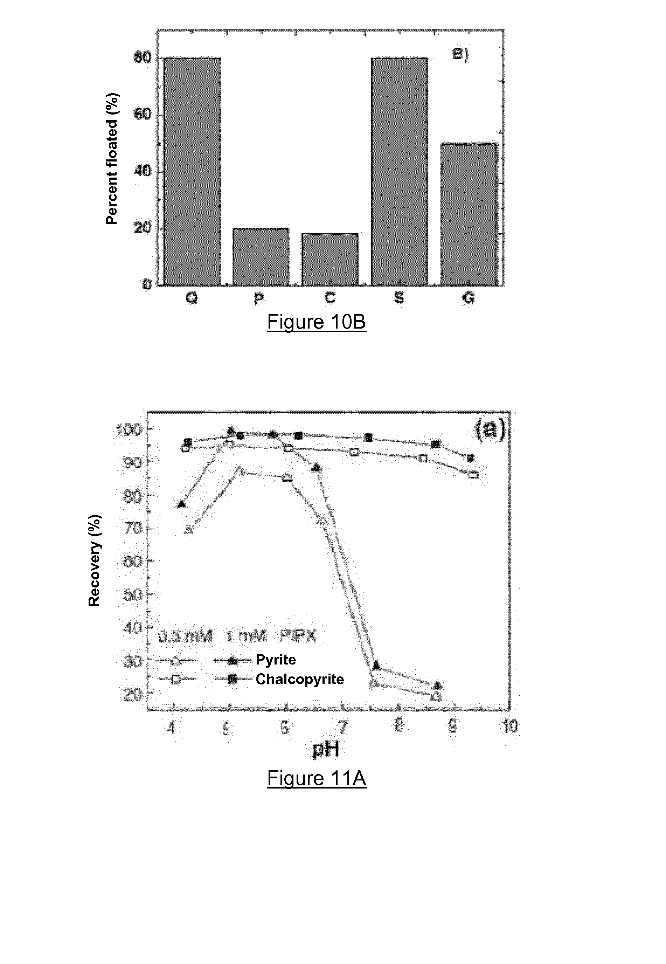

- Figure 10B discloses the results of the test for mineral microflotation of minerals with the proteins.
- Figure 11A discloses the behavior of the flotation of pyrite and chalcopyrite as a function of pH using the PIPX collector.

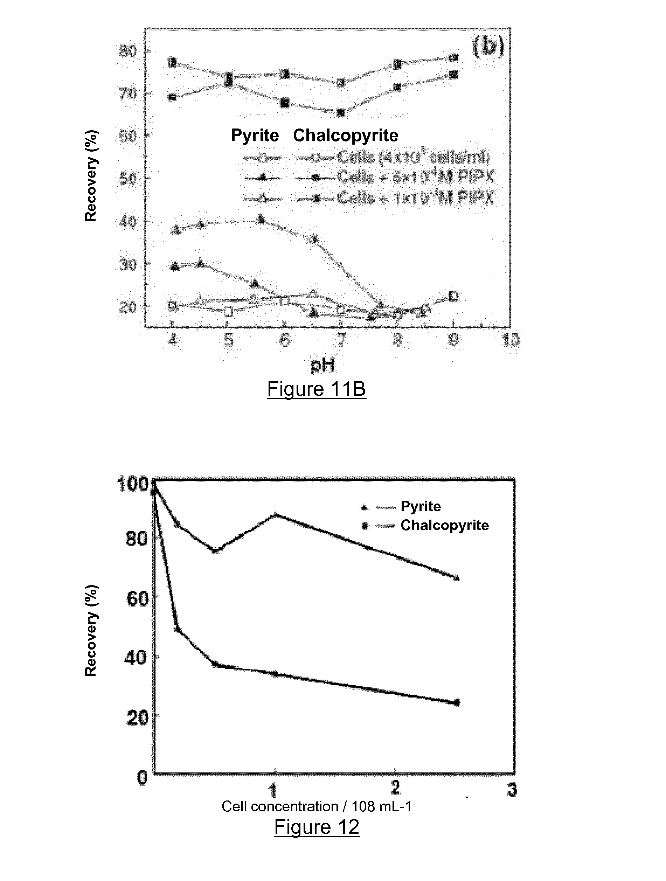

- Figure 11B discloses the effect of cells in the flotation of pyrite and chalcopyrite as a function of pH.
- Figure 12 discloses the result of recovery as a function of pH during flotation of pyrite and chalcopyrite in the presence of cells from *L*. *ferroxidans,* using xanthate as a collector.

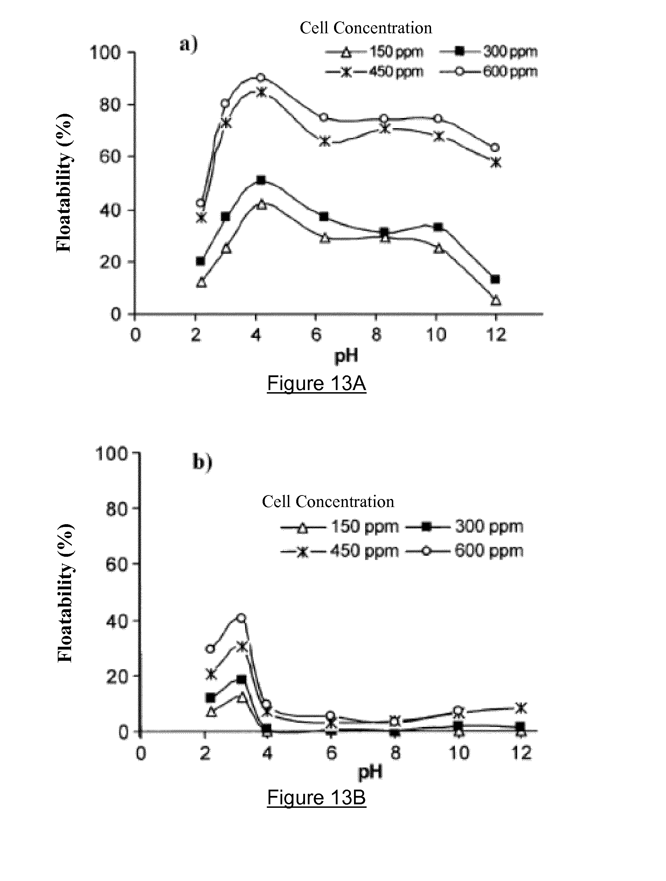

- Figure 13A discloses the results of floatability as a function of pH of hematite in the presence of the bacterium *Rhodococcus opacus.*
- Figure 13B discloses the results of floatability as a function of pH of quartz in the presence of the bacterium *Rhodococcus opacus.*

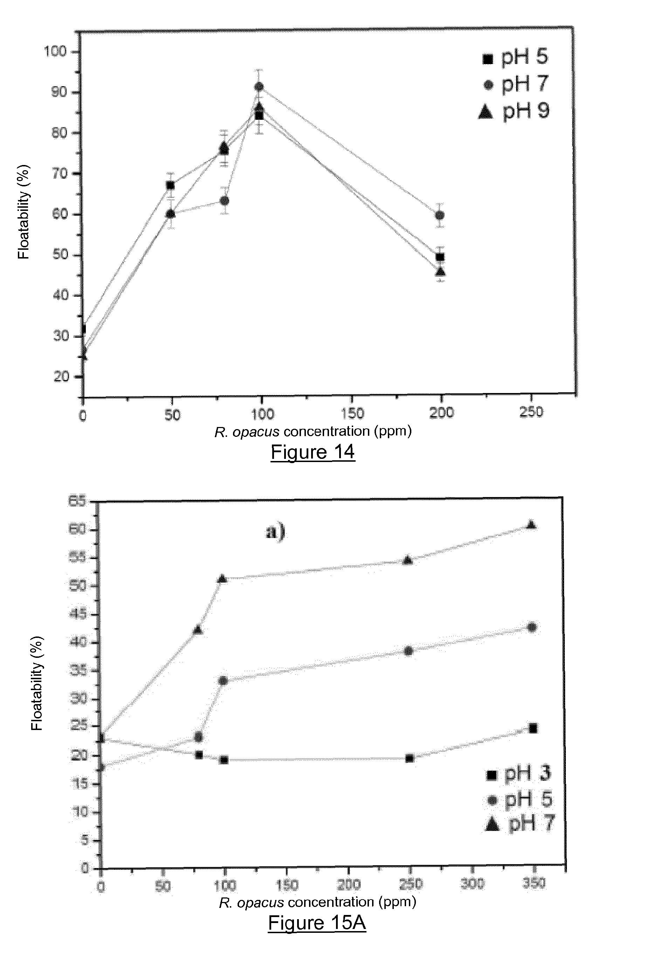

- Figure 14 discloses the results of floatability of magnesite as a function of the concentration of the bacterium *Rhodococcus opacus.*
- Figure 15A discloses the results of floatability of baryte as a function of the concentration of the bacterium *Rhodococcus opacus.*

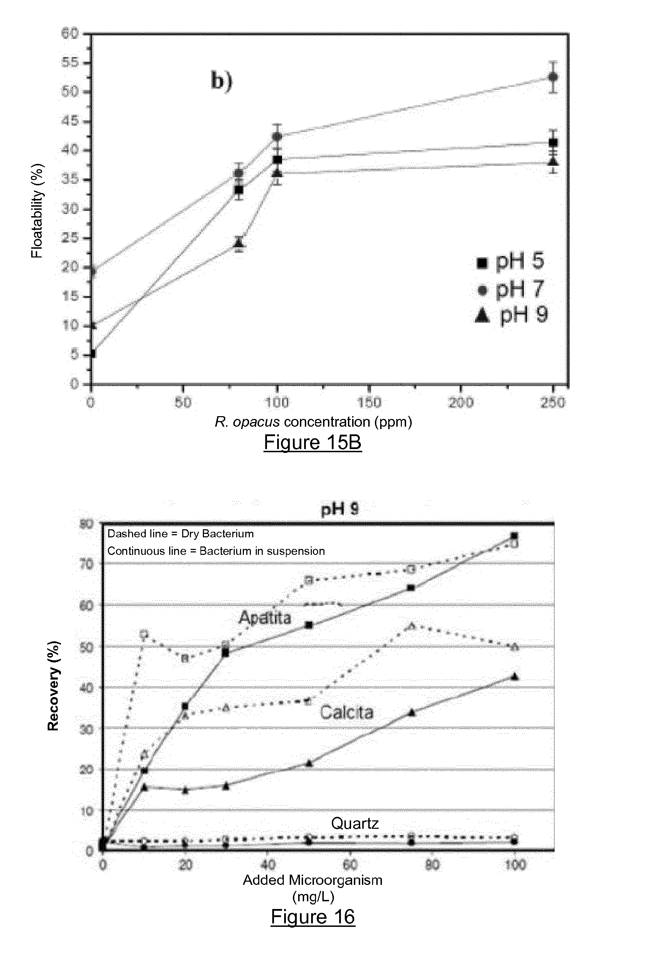

- Figure 15B discloses the results of floatability of calcite as a function of the concentration of the bacterium *Rhodococcus opacus.*
- Figure 16 discloses the rate of recovery of apatite as a function of the concentration of *S. carnosus* in pH equal to 9.

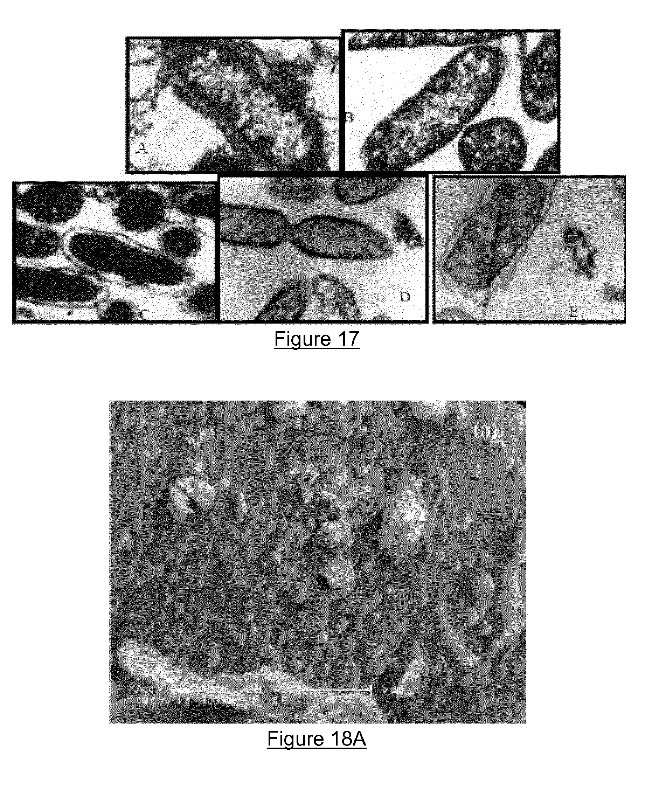

- Figure 17 discloses micrographs of P. polymyxa, developed in the presence of different minerals, made by transmission electron microscopy.
- Figure 18A discloses micrographs of *Desulfovibrio desulfuricans* cells adhered to the surface of hematite, made by scanning electron microscopy.

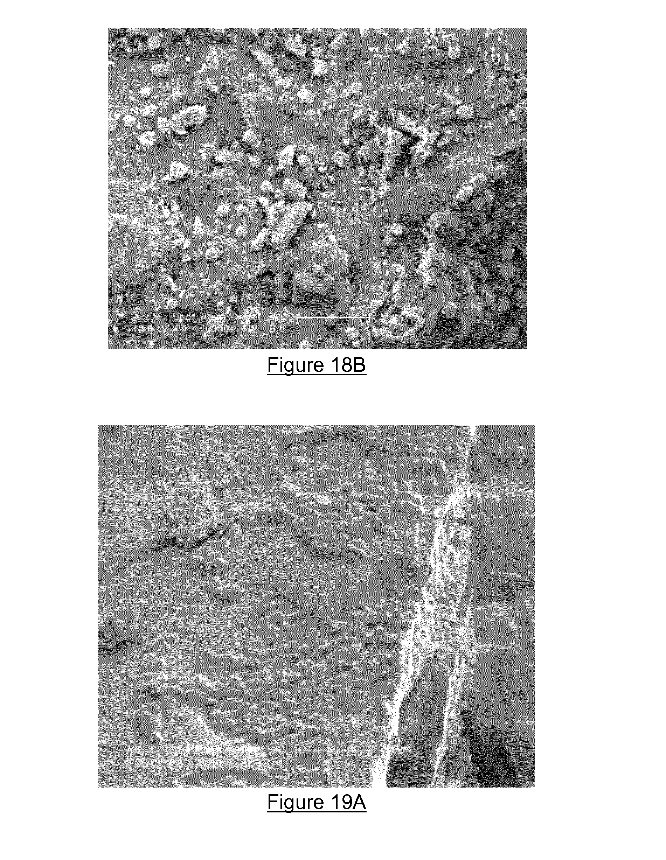

- Figure 18B discloses micrographs of *Desulfovibrio desulfuricans* cells adhered to the surface of hematite, made by scanning electron microscopy.
- Figure 19A discloses micrographs of yeast cells adhered to the surface of calcite, made by scanning electron microscopy.

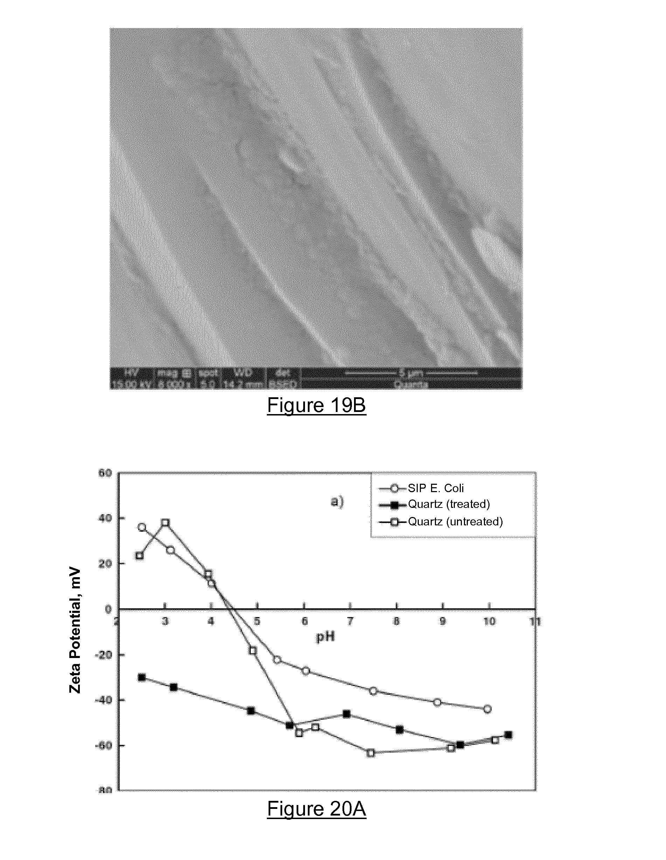

- Figure 19B discloses micrographs of yeast cells adhered to the surface of quartz, made by scanning electron microscopy.
- Figure 20A discloses the zeta potential as a function of pH of quartz in the presence of yeast of the species *E. coli.*

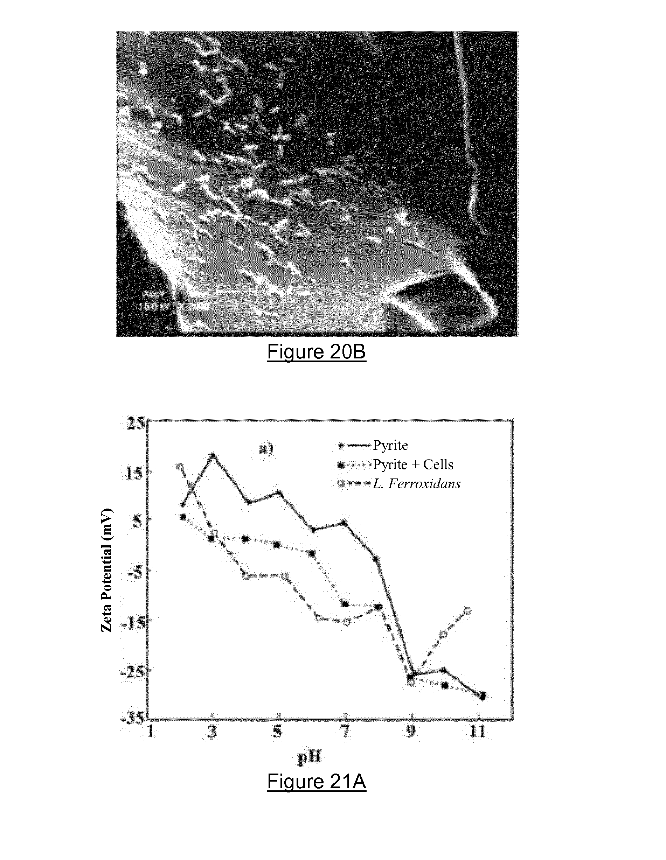

- Figure 20B discloses a micrograph showing the interaction of *E. coli* yeast with the surface of quartz, made by scanning electron microscopy.
- Figure 21A discloses the zeta potential as a function of pH of pyrite before and after interaction with *L. ferroxidans* cells.

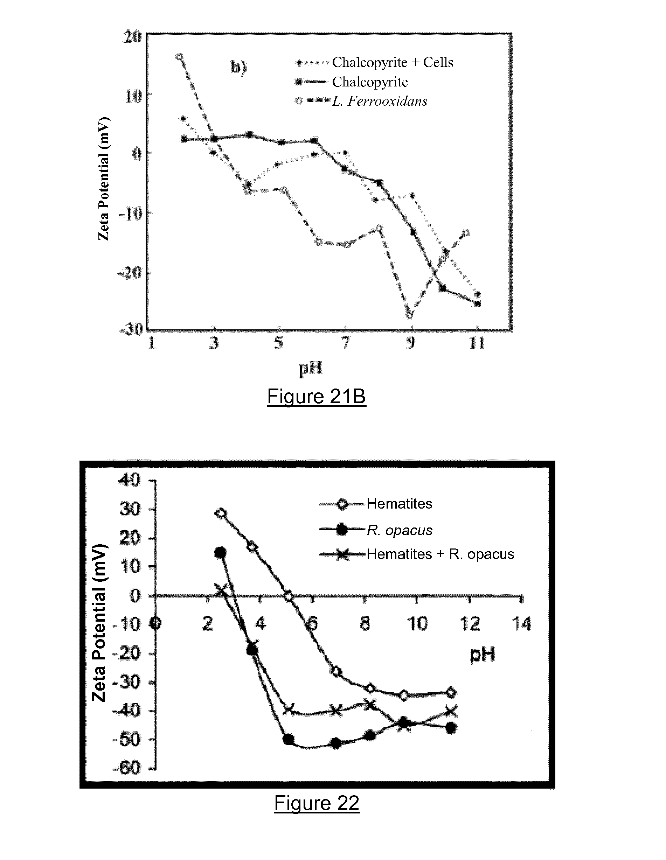

- Figure 21B discloses the zeta potential as a function of pH of chalcopyrite before and after interaction with *L. ferroxidans* cells.
- Figure 22 discloses the zeta potential as a function of pH of hematite before and after interaction with R. *opacus* cells.

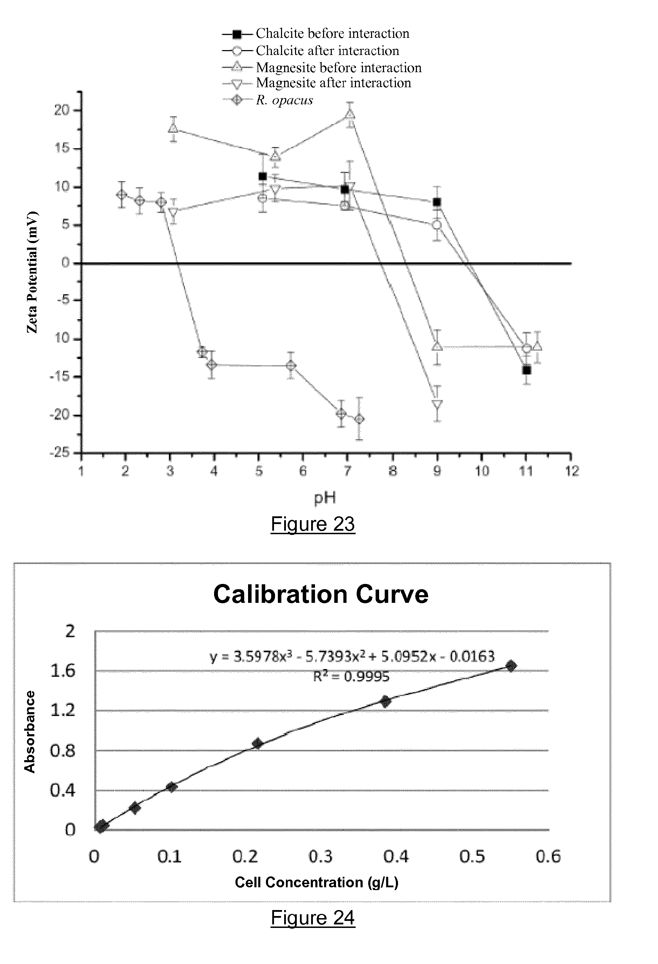

- Figure 23 discloses the zeta potential as a function of pH of calcite and magnesite before and after interaction with R. *opacus* cells.
- Figure 24 discloses the calibration curve obtained by absorbance as a function of the cellular concentration of a *Rhodococcus opacus* suspension.

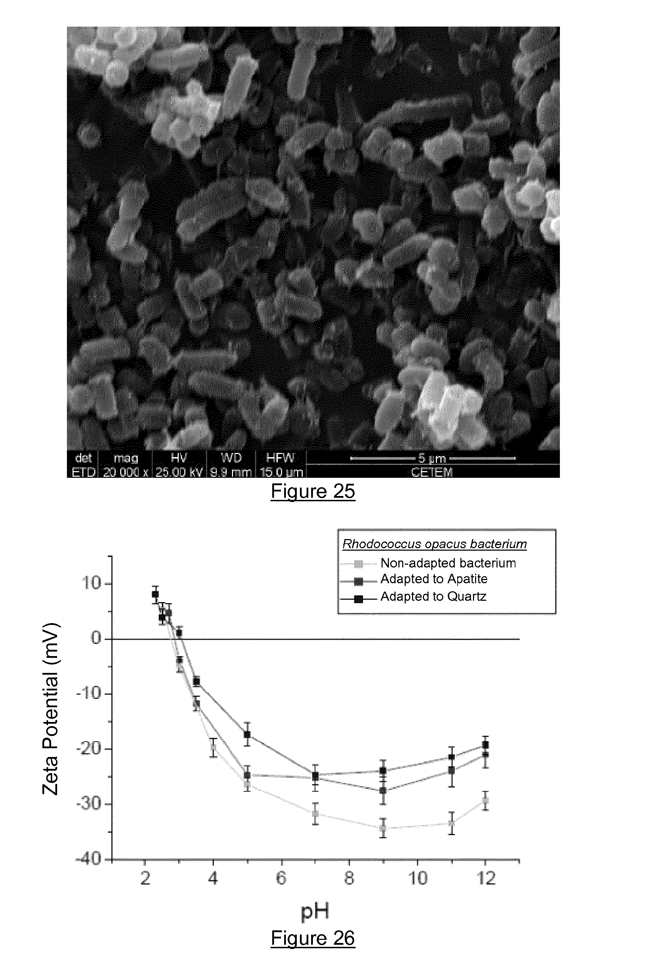

- Figure 25 discloses a micrograph performed by scanning electron microscopy of cells of the bacterium *Rhodococcus opacus.*
- Figure 26 discloses the zeta potential as a function of pH of the bacterium *Rhodococcus opacus* not adapted, adapted to apatite and adapted to quartz using NaCl as electrolyte.

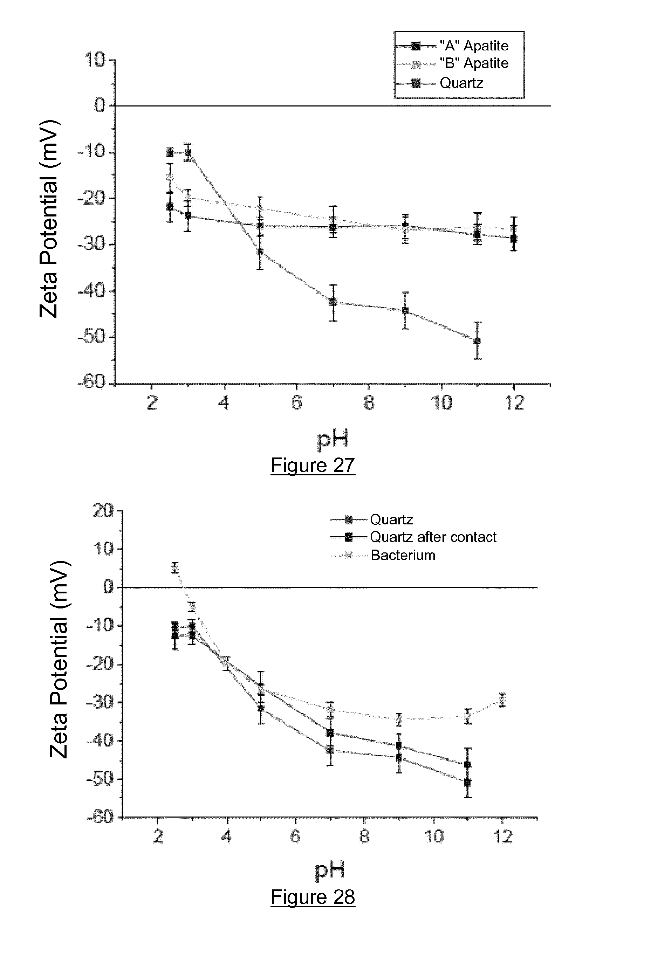

- Figure 27 discloses the zeta potential as a function of pH of the mineral samples of "A" apatite, "B" apatite and quartz using NaCl as support electrolyte.
- Figure 28 discloses the zeta potential as a function of pH of the sample of quartz before and after contact with the bacteria, and the pure bacteria, using NaCl as electrolyte.

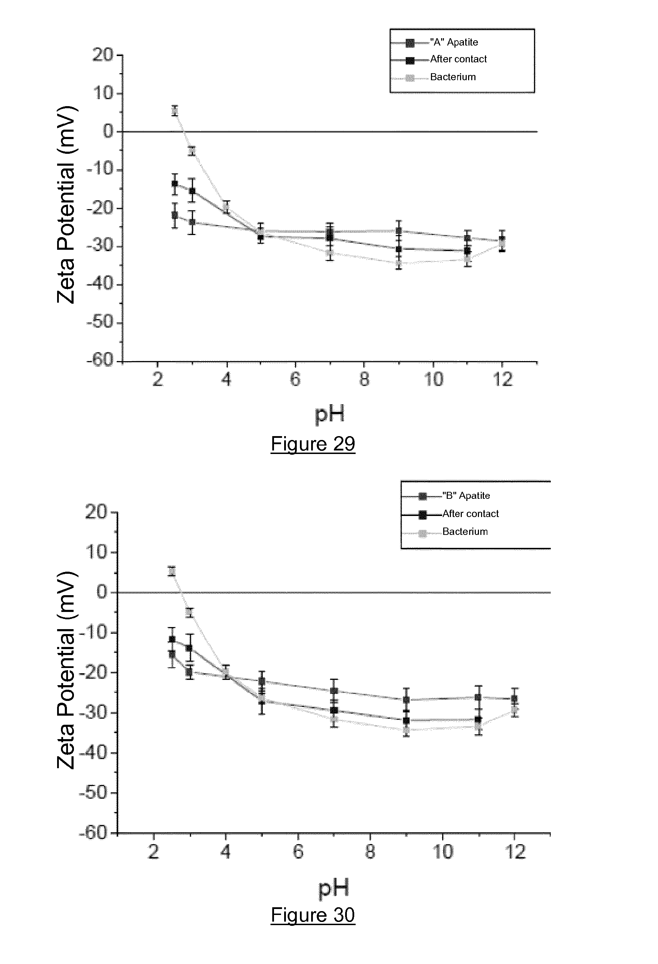

- Figure 29 discloses the zeta potential as a function of pH of "A" apatite before and after interaction with the bacteria, and the pure bacteria, using NaCl as electrolyte.
- Figure 30 discloses the zeta potential as a function of pH of "B" apatite before and after interaction with the bacteria, and the pure bacteria, using NaCl as electrolyte.

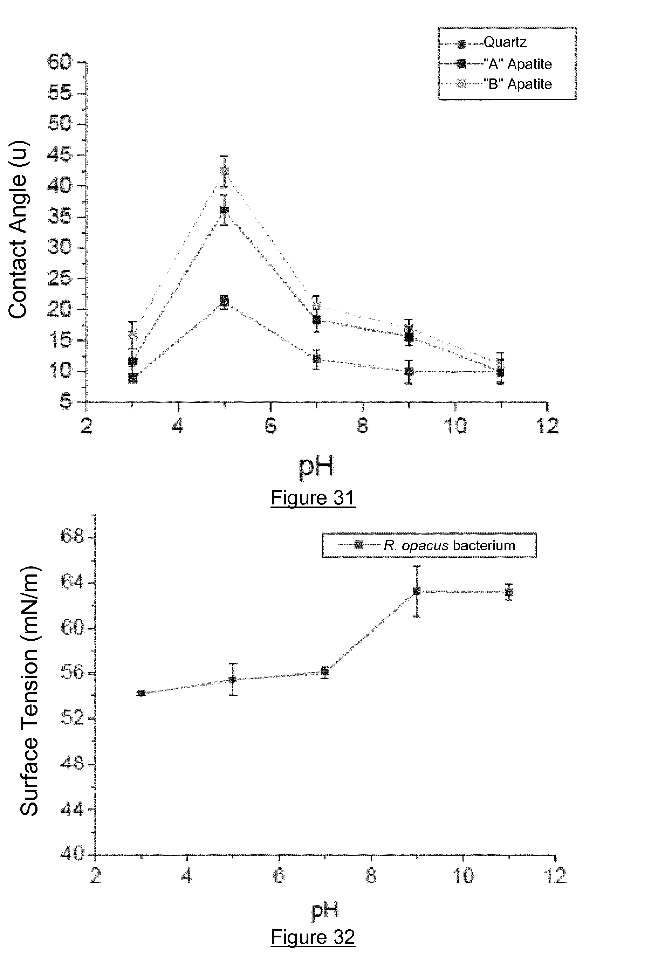

- Figure 31 discloses the contact angle as a function of pH of mineral samples of "A" apatite, "B" apatite and quartz, using a cell suspension of 0.15 g/L concentration, with 5 minutes of contact time.
- Figure 32 discloses the surface tension as a function of the pH of the cell suspension of *Rhodococcus opacus,* in a cell concentration of 0.15 g/L.
- Figure 33 discloses the surface tension as a function of the cell concentration of the bacteria *Rhodocus opacus,* in a pH equal to 5.
- Figure 34 discloses the floatability as a function of pH of "B" apatite, with particle size between 75-106 µm, NaCl as electrolyte and 2 minutes of flotation time.

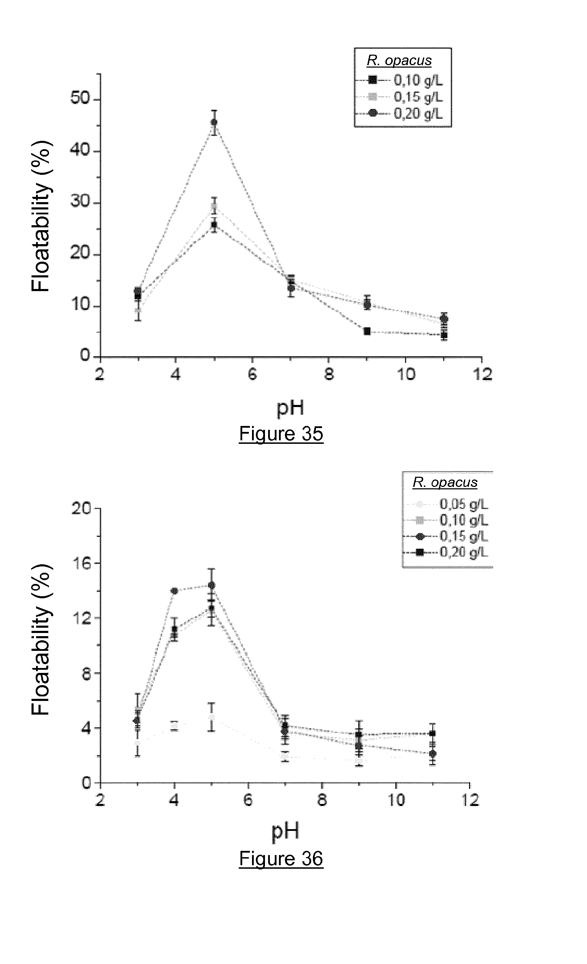

- Figure 35 discloses the floatability as a function of pH of "A" apatite, with particle size between 75-106 µm, NaCl as electrolyte and 2 minutes of flotation time.
- Figure 36 discloses the floatability as a function of pH of quartz, with particle size between 75-106 µm, NaCl as electrolyte and 2 minutes of flotation time.

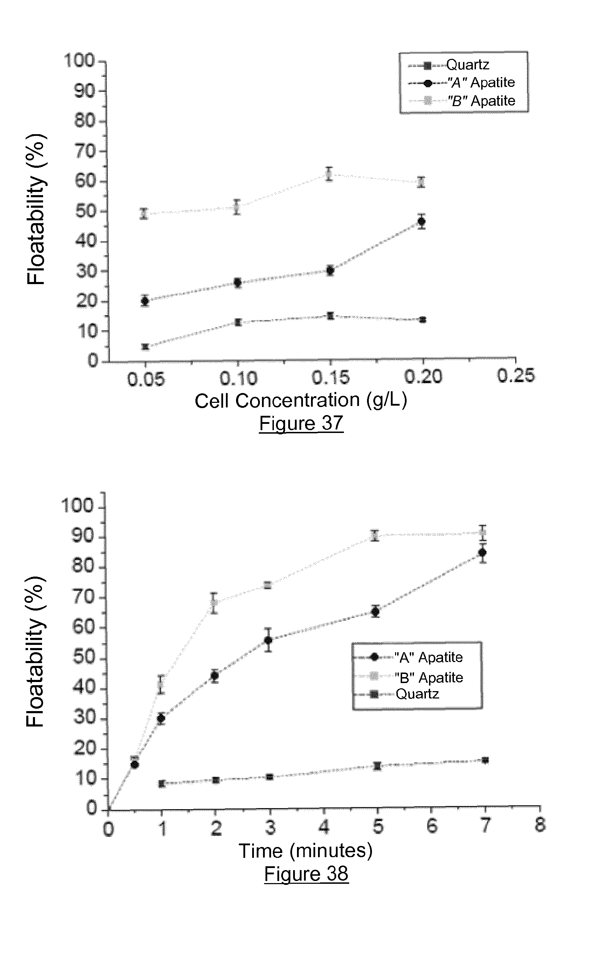

- Figure 37 discloses the cell floatability as a function of cell concentration, in a pH equal to 5, particle size between 75-106 µm, NaCl as electrolyte and 2 minutes of flotation time.
- Figure 38 discloses the cell floatability as a function of time, in a pH equal to 5, particle size between 75-106 µm and using NaCl as electrolyte.

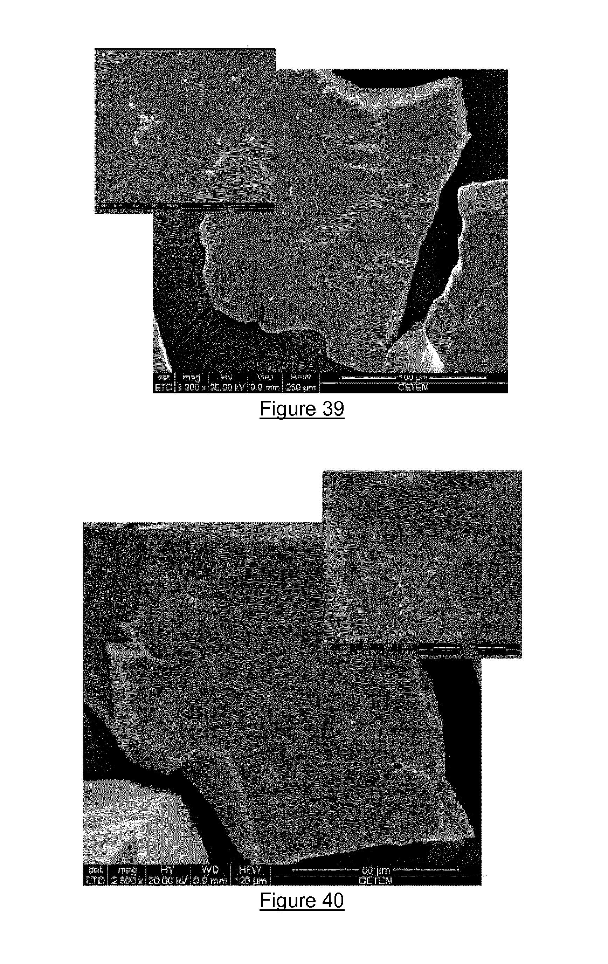

- Figure 39 discloses a micrograph, made by scanning electron microscopy, of cells of *Rhodococcus opacus* adhered on the surface of "B" apatite after the flotation.
- Figure 40 discloses a micrograph, made by scanning electron microscopy, of cells of *Rhodococcus opacus* adhered on the surface of "A" apatite after the flotation.

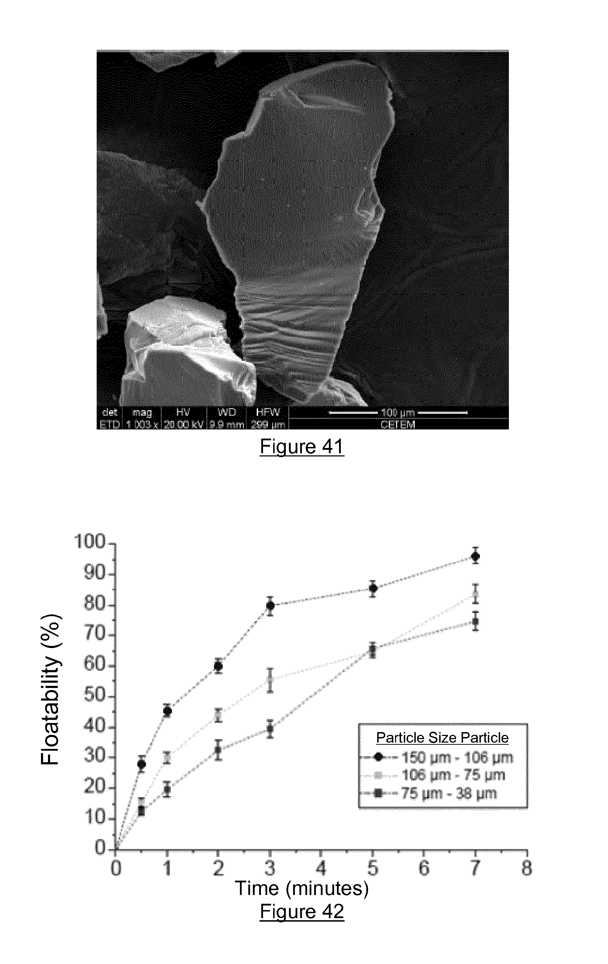

- Figure 41 discloses a micrograph, made by scanning electron microscopy, of cells of *Rhodococcus opacus* adhered on the surface of quartz after the flotation.
- Figure 42 discloses the floatability as a function of time of "A" apatite with different particle sizes, in pH equal to 5, cell concentration 0.2 g/L.

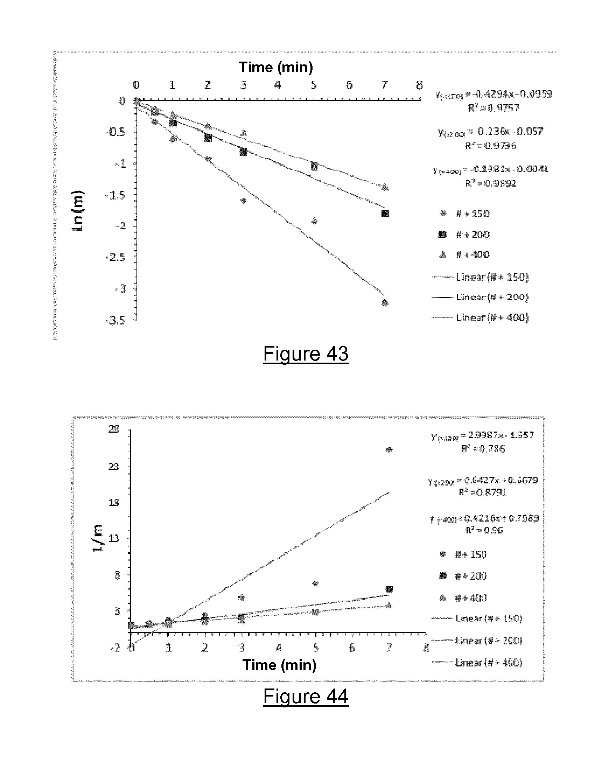

- Figure 43 discloses a first-order model of the floatability of "A" apatite, in pH equal to 5, cell concentration 0.2 g/L.
- Figure 44 discloses a second-order model of the floatability of "A" apatite, in pH equal to 5, cell concentration 0.2 g/L.

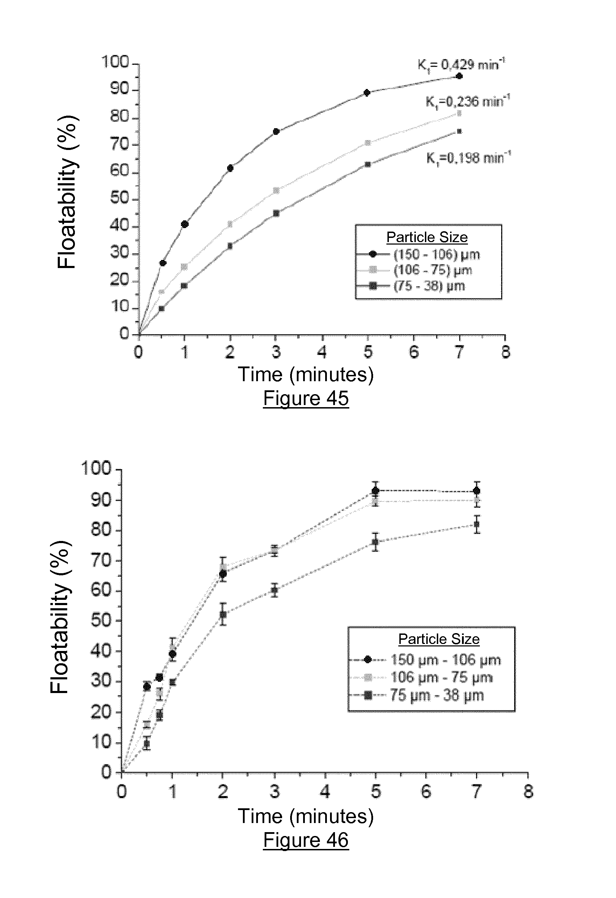

- Figure 45 discloses floatability as a function of time of apatite "A" using the bacterium *Rhodococcus opacus,* obtained from the employment of the first-order model.
- Figure 46 discloses the floatability as a function of time of "B" apatite with different particle sizes, in pH equal to 5, cell concentration 0.15 g/L.

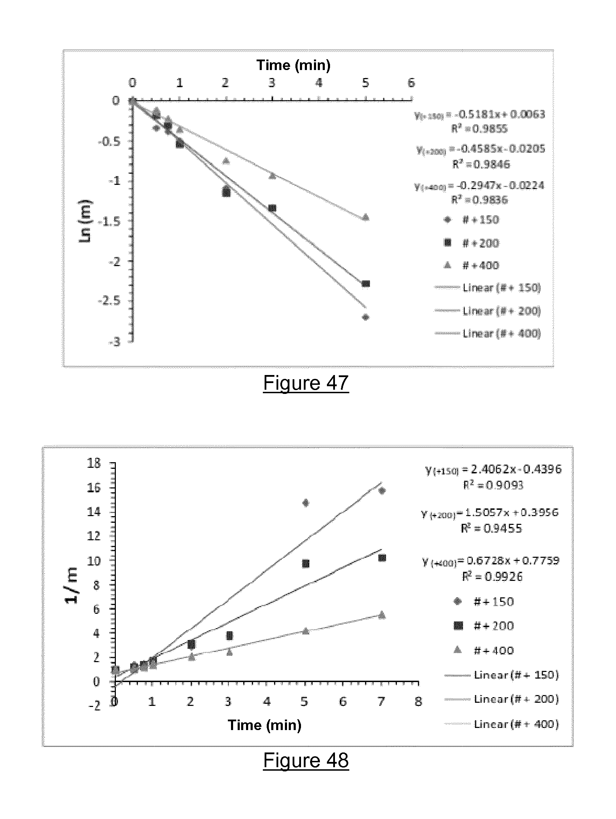

- Figure 47 discloses a first-order model for the floatability of "B" apatite, in pH equal to 5, cell concentration 0.15 g/L.
- Figure 48 discloses a second-order model for the floatability of "B" apatite, in pH equal to 5, cell concentration 0.15 g/L.

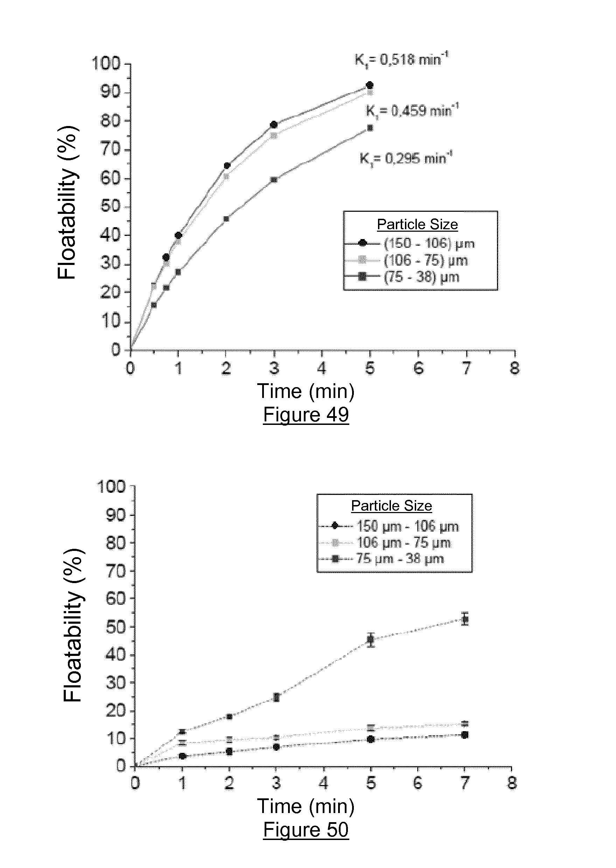

- Figure 49 discloses floatability as a function of time of apatite "B" using the bacterium *Rhodococcus opacus,* with the employment of the first-order model.
- Figure 50 discloses the floatability as a function of time for quartz in different particle sizes, in pH equal to 5, cell concentration 0.15 g/L.

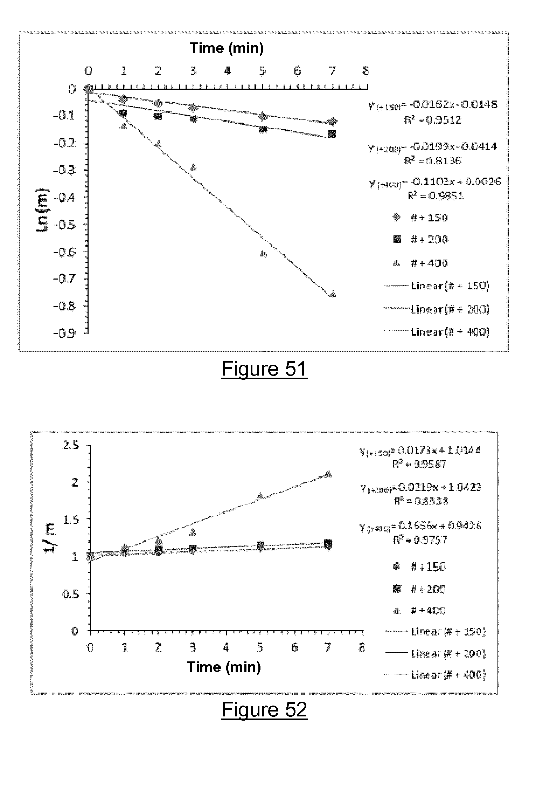

- Figure 51 discloses the first-order model for the floatability of quartz as a function of time, in pH equal to 5, cell concentration 0.15 g/L.
- Figure 52 discloses the second-order model for the floatability of quartz as a function of time, in pH equal to 5, cell concentration 0.15 g/L.

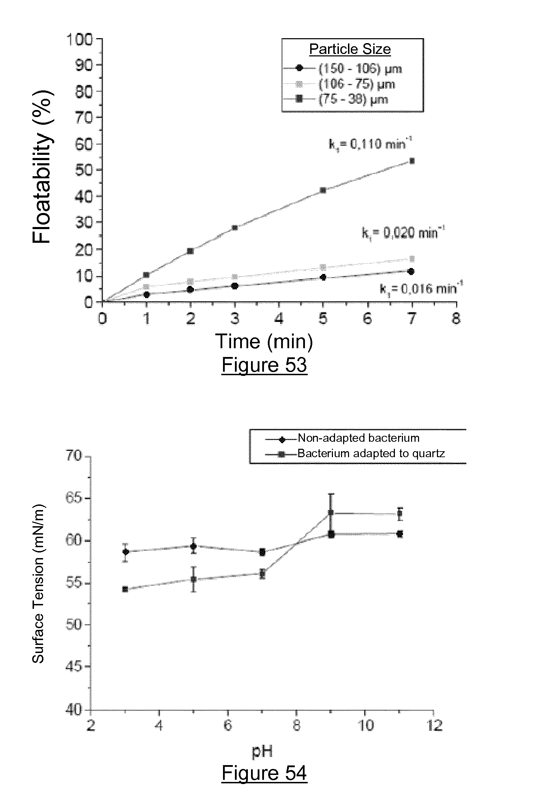

- Figure 53 discloses floatability as a function of time for quartz using the first-order model, for different particle sizes.
- Figure 54 discloses the surface tension as a function of the pH of *Rhodococcus opacus* cells without and with the adaptation to mineral substrate.

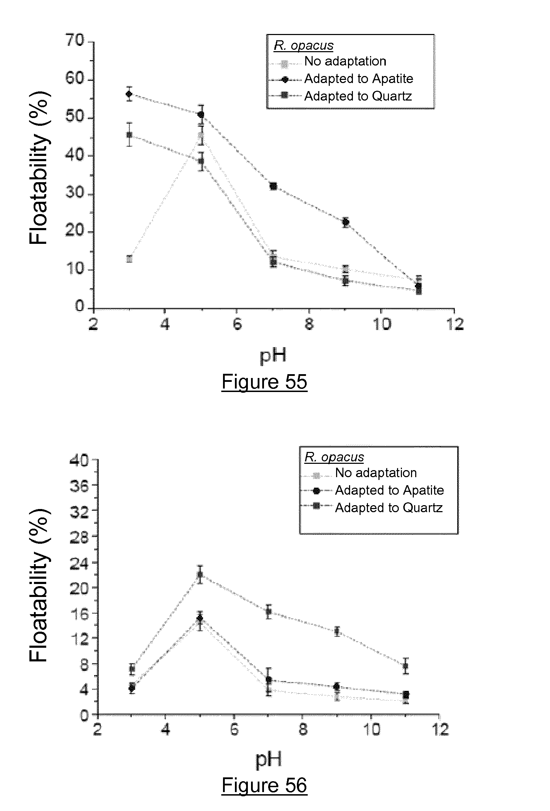

- Figure 55 discloses floatability as a function of pH of the "A" apatite microflotation test, using the bacterium *Rhondococcus opacus* adapted to mineral substrate, in cell concentration 0.20 g/L.
- Figure 56 discloses floatability as a function of pH of the "A" apatite microflotation test, using the bacterium *Rhondococcus opacus* adapted to mineral substrate, in cell concentration 0.15 g/L.

### Detailed Description of the Invention

The examples described herein are intended only to illustrate one of the many ways to accomplish the invention, however, without limiting the scope thereof. Thus, the present invention is based on a new biological adsorbent agent for the bioflotation of minerals constituents of the apatite-quartz system.

### Adsorbent agent

In the present invention, the term agent adsorbent is understood to be the micro-organism able to adhere to the surface of the mineral, thus changing its electrophoretic behavior, this microorganism being chosen among bacteria that make up the genus *Rhodococcus,* more specifically, the species *Rhodococcus opacus.*

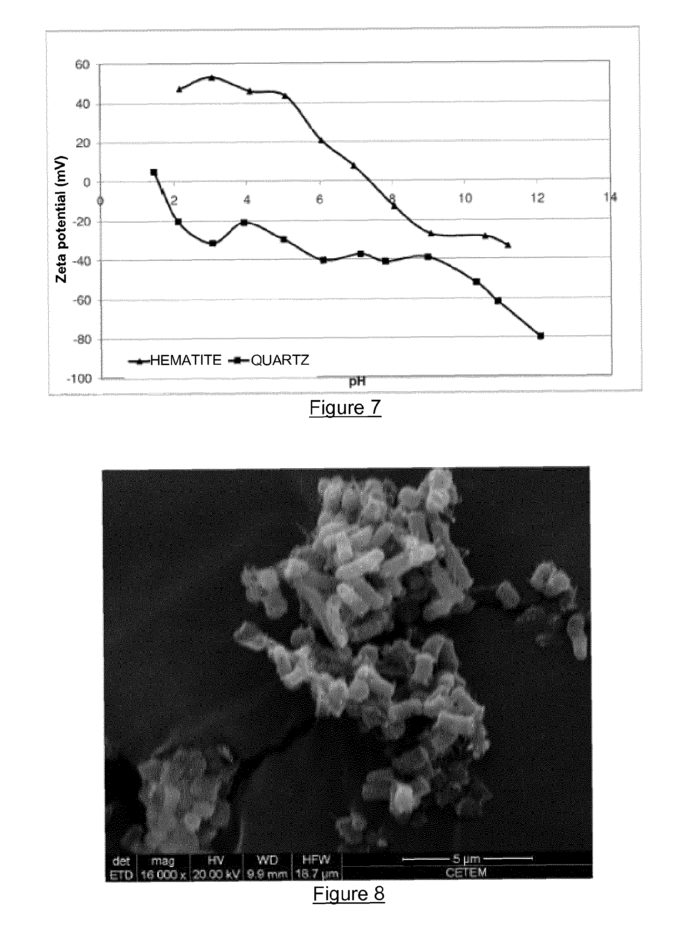

The microorganism *Rhodococcus opacus* (Figure 8) is a bacterium belonging to the genus *Rhodococcus,* unicellular, heterotrophic, Gram-positive and strictly aerobic. The main characteristic of R. *opacus* is the presence of filaments which are responsible for foaming when in aqueous medium. The R. *opacus* cells have in their cell wall various types of components, such as polysaccharides, mycolic acids and lipids which gives an amphipatic character to the surface of the bacterium, presenting a contact angle equal to 72 ± 4 degrees.

The composition of the material belonging to the cell wall of the bacterium R. *opacus* can be seen in Table 1, which shows a high proportion of lipids and carbohydrates associated with the cell wall.

**Table 1 - Composition of the Cell Wall of the Bacterium R. opacus.**

| Material Belonging to the Cell Wall | Concentration (g/L) | Composition (%) |
|---|---|---|
| Proteins | 0.16 | 2.85 |
| Carbohydrates | 0.61 | 10.54 |
| Lipids | 1.92 | 33.33 |
| Cell suspension | 11.52 | - |

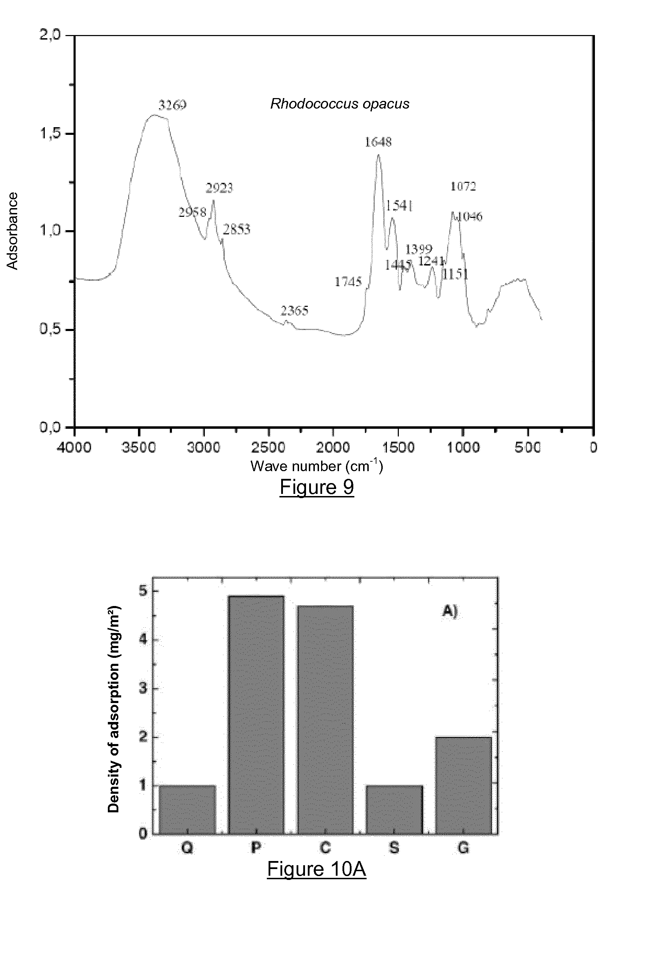

The functional groups present in the cell wall of microorganisms can be determined from the use of infrared spectroscopy. The infrared spectrum of the bacterium displays the peaks of compounds assigned to functional groups of the compounds present in the cell wall. Table 2 shows the ranges of absorbance and the functional groups corresponding to each peak (Figure 9).

**Table 2 - General Band Assignment in Bacterium**

| Wave Number (cm⁻¹) | Corresponding Functional Group |
|---|---|
| 3307 | N-H and O-H vibration from stretching: Polysaccharides and proteins. |
| 2959 | CH₃ asymmetric stretching: Lipids. |
| 2917 | CH₂ asymmetric stretching: Lipids, contribution from proteins, carbohydrates, nucleic acids. |
| 2876 | CH₃ symmetrical stretching: Proteins, contribution from lipids, carbohydrates and nucleic acids. |
| 2857 | CH₂ symmetrical stretching: Lipids, contribution from proteins, carbohydrates, nucleic acids. |
| 1739 - 1744 | Ester C=O stretching: Lipids, triglycerides. |
| 1657 | Amide I (Protein C=O stretching). |
| 1541 | Amide II (Protein N-H band, C-N stretching). |
| 1452 | CH₂ bindings: Lipids. |
| 1391 | COO⁻ symmetric stretching: chains amino acids, fatty acids. |
| 1236 | PO₂⁻ asymmetric stretching: Nucleic acids with contribution of phospholipids. |
| 1152 | CO-O-C asymmetric stretching: Glycogen and nucleic acids. |
| 1080 | PO₂⁻ symmetric stretching: nucleic acids and phospholipids, C-O stretching: glycogen. |
| 969 | C-N⁺-C stretching: Nucleic acids. |
| 958 | Xylo-oligosaccharides. |
| 859 | Type N sugar. |
| 801 | P-O stretching: nucleic acids. |
| 728 | Dipicolinic acid (DPA). |
| 703 | Dipicolinic acid (DPA). |
| 550 | Glycogen. |

If the bacteria are adapted to different substrates, namely, some mineral, there could be a change in the rate of production of metabolic products, thus altering the functional groups present and as a result the obtaining of a distinct and even improved response in mineral processing. Thus, the bacterium R. *opacus* was adapted to the presence of apatite and quartz.

### Minerals

The present invention considers as minerals for bioprocessing, the system composed of apatite-quartz. The generic chemical formula of apatite is: Ca₅(PO₄)₃(OH,Cl,F) being named as hydroxyapatite, chloroapatite or fluorapatite depending on the ion present in the structure as shown in Table 3.

**Table 3 - Some Examples of the Apatite Group (D/L)₃(D'/L')₂(TO₄)₃X**

| Apatite Subgroup - (PO₄) | |
|---|---|
| Chloroapatite | Ca₅(PO₄)₃Cl |
| Fluorapatite | Ca₅(PO₄)₃F |
| Pyromorphite | Pb₅(PO₄)₃Cl |
| Apatite-Strontium | Sr₅(PO₄)₃(OH) |

As previously shown, there can be found different types of apatite mineral and, consequently, the performance in view of beneficiation processes will be different for each one. Therefore, it is of the utmost importance to obtain the knowledge of the physico-chemical properties of these minerals such as crystalline structure, mineral composition, solubility, zeta potential and acting mechanisms of adsorption for the study of the performance of flotation.

In the literature, a wide variation in the value of the isoelectric point of apatite can be observed. Finding PIE values from 2 to 8, the reason for this difference depending mainly on the origin and the same.

The knowledge of electrokinetic characteristics of a mineral in aqueous solution being important, because it helps to elucidate the mechanisms involved in adsorption of reagents of flotation on the surface of the mineral, as well as performs a very important role in the response to the process of mineral concentration (flotation).

### Composition (%)

The composition according to the present invention comprises preferably a cell suspension, where this presents a strain of *Rhodococcus opacus* inactivated after the phase of cell growth, along with the supporting electrolyte. This electrolyte will act on the re-suspension of bacteria after the centrifugation step, thus allowing a homogeneous solution of cell concentrate. The cell electrolyte used comprises a saline solution, so that it does not change the pH value intended for the present invention, and it is possible to adopt the use of sodium chloride as support electrolyte.

The method of bioflotation preferably used by the test can be conducted in a modified Hallimond tube. This presents other equipment attached in order to perform the test, such equipment are: rotameter, for the measurement of air flow; bubble meter, to calibrate the rotameter; magnetic stirrer, to maintain the mineral particles in suspension; vacuum pump-compressor, to maintain the required air to the Hallimond tube.

### Example 1. Preferred Embodiment

Initially, the samples of apatites are submitted to the step of comminution followed by sieving, this way, the product obtained of these steps is classified in different grain size fractions. The samples are then characterized in different experimental steps, eletrophoretic measures, contact angle, X-ray diffraction, X-ray fluorescence, scanning electron microscopy and transmission.

After the characterization, the samples of apatite are subjected to washing with a 0.01 mM hydrochloric acid solution, subsequently washed quickly with Milli-Q water several times until the pH value of the effluent reaches the value of the initial pH of water, and subsequently they are dried and stored in desiccator up to the time when they are to be used in experimental trials. The samples of quartz were washed with 0.01 M KOH. Shortly thereafter, the same procedure done in the apatite minerals was carried out.

In parallel, there is the preparation of bacterial concentrate and conditions of culture thereof of the bacterial species called *Rhodococcus Opacus.*

Firstly, all glassware used, as well as the different culture media, were sterilized by autoclaving at 1 atm. pressure and 121 °C for 20 minutes. The bacterial strain was grown on solid medium - composition shown in Table 4 - in Petri dishes and taken to incubation until the bacteria colonies were identified.

**Table 4 - Culture Medium Used in Bacterial Culture**

| Component | Solid (g/L) | Liquid (g/L) |
|---|---|---|
| Glucose | 4 | 10 |
| Peptone | 5 | 5 |
| Malt extract | 10 | 3 |
| Yeast extract | 4 | 3 |
| CaCO₃ | 2 | - |
| Hagar | 12 | - |
| pH | 7.2 | 7.2 |

Subsequently, the bacterium was sub-cultivated in liquid culture medium - composition shown in Table 5 - in 250 mL Erlenmeyers and taken to incubation in a rotary Shaker (CIENTEC CT-712) at a temperature of 28°C for 24 hours.

**Table 5 - Grain Size Fractions of Minerals for Each of the Tests Performed**

| Experiment | Particle size |
|---|---|
| Zeta potential tests | < 38 µm |
| Microflotation experiments | (150 - 105) µm |
| | (105 - 75) µm |
| | (75 - 38) µm |
| Contact angle measurements | (0.5 × 0.5 × 1.0) cm |

After the last growth, the cell suspension was centrifuged at 3300 g for 8 minutes; the centrifugation concentrate, composed by cells of the bacterium, was washed three times with deionized water, and re-suspended in a 1 mM NaCl solution; finally, the concentrated suspension obtained was sterilized in autoclave to inactivate the bacteria present. This final concentrate is the biomass used as bioreagent in the development of the work.

The cellular concentration of the bacterial suspension was determined by optical density in a spectrophotometer (UV-Spectrophotometer, UV-1800, Shimadsu) at wavelengths specific for the bacterium (λ= 620nm). We performed a calibration of the dry weight of biomass against the optical density of the suspensions in the same wavelength. The dry weight of biomass was determined after filtration through Millipore system in vacuum using 0.45 µm cellulose membrane (Millipore, USA) and finally dried in oven at 160°C.

In order to confirm the selective behavior displayed by a bacterial strain after adaptation to a mineral substrate, the R. *opacus* strain suffered adaptation to the presence of mineral samples such as quartz and "A" apatite. The adaptation of the bacterium was performed during the development of the bacterial cells under the same conditions of cultivation and using the standard liquid culture medium in the presence of the mineral with a concentration of 5% (w/v) in 3 sub-cultures in succession.

The measurements of zeta potential for bacteria as well as for the mineral samples were determined in a Zeta meter system +4.0 type micro electrophoresis equipment. Thus, zeta potential assays were performed to evaluate the influence of the interaction of bacterial cells on the surface of mineral species. In this case, a pre-conditioning of mineral solutions was performed with a cell suspension of known concentration during 10 minutes. After this period, the supernatant was used in the measurements. Different pH values were evaluated in preconditioning, using as an electrolyte a 1mM NaCl solution. To ensure the accuracy of the measurement, the mean of 20 values and the standard deviation value were taken.

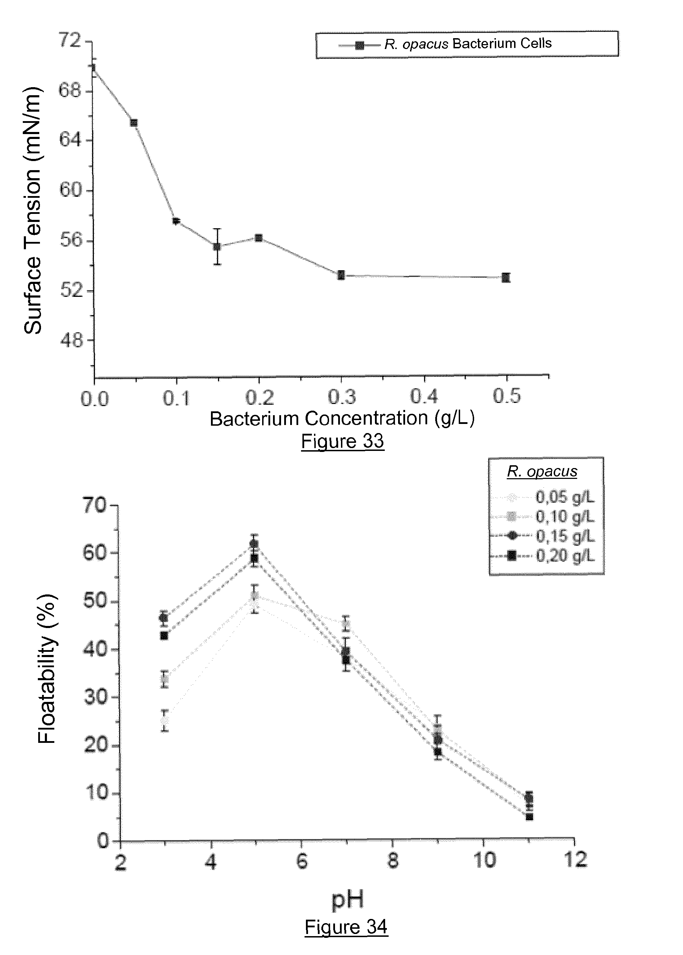

To evaluate the possible change in hydrophobicity of the surface of minerals after the adhesion of bioreagent, the contact angle values of the mineral samples will be measured before and after interaction with the bacterium. A Rame Hart-inc model 100-00-115 goniometer will be employed (Fig.33).

To measure the contact angle values of mineral samples, polished sections of minerals measuring 0.5 x 0.5 x 1.0 cm (Fig.34) were molded with epoxy resin. The top of the surface of each sample was then carefully polished until it reached the suspension of diamond (1µm). The surfaces of the sections of each mineral were carried to ultrasound bath for 2 minutes and immediately washed with jets of deionized water to remove small particles adhered.

The packaging of the mineral surface was performed with cell suspension of bacteria with a known concentration (0.1 g.L -1), NaCl 0.001 M and with different pH values, values adjusted with aliquots of HCl and NaOH. Drops of the cell suspension were deposited on the surface of the minerals and left to rest for 10 minutes. Then the samples were washed with 0.001 M NaCl solution to remove the non-adhered cells. They were later submerged in the same solution with the same pH value of the conditioning. Finally, air bubbles were released with 5µm diameter size on the surface, the measurements of contact angle being performed in a goniometer, using the captive bubble method.

Before each contact angle measurement at the polished sections, a polishing with diamond suspension and ultrasound bath were performed. Subsequently, the sections were washed several times, and kept immersed in Milli-Q water for short periods of time, before proceeding to a new test. The cleaning of surfaces was verified by previously measuring the contact angle in Milli-Q water, presenting value equal to zero, to the surfaces of minerals.

The microflotation tests were conducted in a modified Hallimond tube. To this end, a rotameter was needed to measure the air flow rate, a bubble meter to calibrate the rotameter, a magnetic stirrer to maintain the mineral particles in suspension, a vacuum pump-compressor to maintain the required air, and the Hallimond tube. Before performing the tests the rotameter must be calibrated to ensure an air flow rate of 15 mL.min-1.

## Claims

1. ADSORBENT AGENT, wherein it is used for adsorption on the surface of the apatite-quartz system and comprises at least one bacterium selected from the group comprising species of the genus *Rhodococcus.*

2. ADSORBENT AGENT, according to claim 1, wherein the bacterium is *Rhodococcus opacus.*

3. ADSORBENT AGENT, according to claim 2, wherein the bacterium *Rhodococcus opacus* is adapted to the medium.

4. ADSORBENT AGENT, according to claim 1, wherein the apatite is from the fluoroapatite subgroup.

5. BIOFLOTATION COMPOSITION, wherein it is used in an apatite-quartz system and comprises:
a. at least one adsorbent agent comprising at least a bacterium selected from the group comprising the species of the genus *Rhodococcus;* and
b. an acceptable carrier.

6. COMPOSITION, according to claim 5, wherein the carrier is a support.

7. COMPOSITION, according to claim 6, wherein the support consists of a saline solution.

8. COMPOSITION, according to claim 7, wherein the saline solution comprises a sodium chloride solution.

9. PROCESS FOR BIOFLOTATION OF THE APATITE-QUARTZ SYSTEM, wherein it comprises adding to an apatite-quartz system an adsorbent composition comprising at least one adsorbent agent comprising at least a bacterium selected from the group comprising the species of the genus *Rhodococcus.*

10. PROCESS FOR BIOFLOTATION OF THE APATITE-QUARTZ SYSTEM, according to claim 9, comprising the steps of:
a. adding the apatite-quartz system in an adsorbent composition comprising at least one adsorbent agent comprising at least a bacterium selected from the group comprising the species of the genus *Rhodococcus;*
b. allowing the adsorbent agent to contact the solution comprising the apatite-quartz system in a Hallimond tube, for flotation during two minutes; and
c. collecting the floated mass comprising quartz and the adsorbent agent.

11. PROCESS FOR BIOFLOTATION OF THE APATITE-QUARTZ SYSTEM, according to claims 9 or 10, wherein the bacteria of the genus *Rhodococcus* comprises the species *Rhodococcus opacus.*

12. PROCESS FOR BIOFLOTATION OF THE APATITE-QUARTZ SYSTEM, according to claim 11, wherein the bacterium *Rhodococcus opacus* is a bacterium adapted by being previously exposed to minerals.

13. PROCESS FOR BIOFLOTATION OF THE APATITE-QUARTZ SYSTEM, according to the preceding claims, wherein the apatite mineral comprises the fluorapatite subgroup.

14. PROCESS FOR BIOFLOTATION OF THE APATITE-QUARTZ SYSTEM, according to the previous claims, wherein the time of flotation is about two minutes and the pH of flotation is acid.
